# EUROPEAN PATENT SPECIFICATION

(11) **EP 3 952 801 B1**
(45) Date of publication and mention of the grant of the patent: **25.11.2026**
(21) Application number: 20787280.5
(22) Date of filing: 03.04.2020
(51) Int. Cl.: A61F 5/01

(54) **A LOWER LIMB ORTHOSIS**
ORTHESE DER UNTEREN GLIEDMASSEN
ORTHÈSE DE MEMBRE INFÉRIEUR

(30) Priority: 08.04.2019 US 201962919988 P
(43) Date of publication of application: 16.02.2022
(73) Proprietor: LB2 Technologies, LLC, Destin FL 32541 (US)
(72) Inventor: MADDEN, William Byrne, Destin, FL 32541 (US); CHEN, Leo, Chuen, Destin, FL 32541 (US)
(74) Representative: Stöckeler, Ferdinand
(86) International application number: PCT/IB2020/000310
(87) International publication number: WO 2020/208423

(56) References cited:
- US-A- 5 718 673
- US-A- 5 776 090
- US-A1- 2007 244 420
- US-A1- 2014 288 475
- US-A1- 2015 305 911
- US-A1- 2015 320 581
- US-B1- 6 692 454

## Description

### FIELD

This application relates generally to orthoses for lower limbs.

### BACKGROUND

In a normal gait cycle, the leg is first extended, placing the heel in contact with the ground and with the ankle in dorsiflexion. Then weight is transferred onto the foot which causes the foot to lie flat on the ground. Next, the weight is transferred from the heel region to the ball of the foot. Finally, the foot rotates from the ankle in a motion known as plantarflexion. Plantarflexion causes the heel to lift off the ground at the end of a stride. When the foot lifts off the ground, the normal gait cycle starts over again as the foot approaches heel strike.

A number of pathological conditions may interfere with a subject's ability to perform dorsiflexion or plantarflexion completely or correctly. For example, a partial or complete block of nerve function to the lower leg may hinder plantarflexion and/or dorsiflexion. Conditions such as diabetic nerve pain, drop foot, plantar fasciitis, traumatic nerve injury and demyelination, or iatrogenic nerve injury may cause abnormal foot and ankle movement during the gait cycle. Subjects experiencing these conditions may be unable to walk, run, or ride a bicycle normally. Walking with an altered gait or limp can contribute to pain, dysfunction, and deterioration of the knees, hips, and spine.

One solution is to provide electrical stimulation to foot and/or ankle muscles at appropriate times during ambulation. A number of orthoses incorporate electrodes that emit an electrical current causing muscles to contract even in the absence of a nerve signal. The electrical stimuli may be coordinated in response to signals from pressure sensors in the sole of the orthosis. However, these orthotic devices are expensive to manufacture and are limited by the battery life of the device. Additionally, the subject must carry a cumbersome controller that typically houses a bulky battery and a microprocessor. Another common solution is an ankle foot orthosis that consists of a posterior leg portion that extends into a plantar plate that blocks plantarflexion.

Document US 2015/305911 A1 describes a modular orthosis system for an orthosis of the lower limb, which should facilitate an individual adjustment to the respective patient and to his loss of function to be quickly adjusted to different situations.

Document US 5 776 090 A relates to the general art of orthopedic braces, and to the particular field of treating Plantar Fasciitis.

Document US 2015/320581 A1 relates to an orthopedic device for a lower limb of a human, comprising a leg part.

Document US 2007/244420 A1 relates to orthopedic braces and, in particular, to ankle braces, foot braces, ankle supports, or foot supports.

### SUMMARY OF THE INVENTION

The invention is set out in the appended set of claims.

It is an aspect of the present invention to provide an orthosis that permits plantarflexion of the foot. The orthosis has a shell portioned and dimensioned to be in close proximity to the foot and leg of a subject. The shell has:
(a) a leg portion that is fitted over the anterior surface of the leg;
(b) a foot portion that is fitted over the dorsal surface of the foot and adapted to impede dorsiflexion of the foot; and
(c) a heel portion that can lift the heel when a force is applied to the leg portion.

The shell of the orthosis is a single rigid form.

The shell of the orthosis may be molded to the foot and leg of the subject.

The orthosis is formed to support the foot in a specific angle of dorsiflexion.

The orthosis may have a component for securing the leg portion to the subject's leg, and particularly the lower leg, and subsequently releasing the leg portion from the subject's leg.

The orthosis may have a calf member fitted over the calf which may be secured to and subsequently released from the leg portion.

Some or all portions of the orthosis may be suitable for wearing inside footwear or under legwear such as shoes, boots, pants and/or trousers.

It is another aspect of the present invention to provide an orthosis that permits plantarflexion and has a shell portioned and dimensioned to be in close proximity to the foot and leg of a subject. The shell has:
(a) a leg portion that is fitted over the anterior surface of the lower leg and can be secured to the leg and subsequently released.
(b) a foot portion fitted over the dorsal surface of the foot that impedes dorsiflexion of the foot.
(c) a heel portion that can lift the heel when an anterior force is applied to the leg portion.

The shell is comprised of a single rigid form.

The orthosis may be molded at 90 degrees of dorsiflexion of the foot.

The orthosis may include a calf member fitted over the posterior of the leg which may be secured to and subsequently released from the leg.

The foot portion of the orthosis includes a plurality of toe portions fitted over the dorsal surface of a plurality of toes.

Some or all of the orthosis may be suitable for wearing inside footwear or legwear such as shoes, boots, and/or trousers.

These together with other aspects and advantages which will be subsequently apparent, reside in the details of construction and operation as more fully hereinafter described and claimed, reference being had to the accompanying drawings forming a part hereof, wherein like numerals refer to like parts throughout.

### BRIEF DESCRIPTION OF THE DRAWINGS

FIG. 1 shows a medial view of an orthosis 1 in accordance with various aspects and embodiments of the invention.
FIG. 2 shows a lateral view of the orthosis 1.
FIG. 3 shows a top view of the orthosis 1.
FIG. 4 shows a front view of another orthosis 14.

### DETAILED DESCRIPTION OF THE PREFERRED EMBODIMENTS

An orthosis for a lower limb is provided. In one aspect, the orthosis is comprised of a shell that is portioned and dimensioned so as to be in close proximity to an anatomical limb. The shell has a leg portion, a foot portion, and a heel portion, the leg portion being fitted over the anterior surface of the leg, the foot portion being fitted over the dorsal surface of the foot, and the heel portion being fitted over the surface of the heel and adapted to lift the heel when AN ANTERIOR force is applied to the leg portion. The orthosis impedes dorsiflexion and permits plantarflexion. In this disclosure, the term "impede" means that the orthosis may permit some degree of dorsiflexion, but dorsiflexion will be controlled or limited by the orthosis.

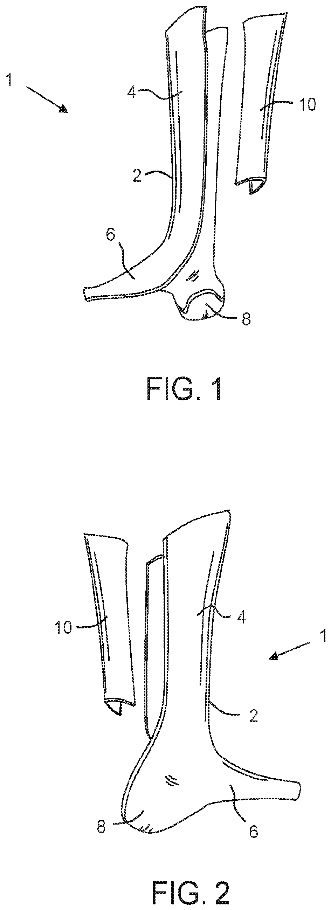

Referring to FIG. 1, an orthosis 1 is generally shown. The orthosis comprises a shell 2 having a leg portion 4, a foot portion 6, and a heel portion 8.

The foot portion 6 is fitted over the dorsal surface of the foot and is adapted to impede dorsiflexion of the foot. The dorsal surface of the foot covered by the foot portion 6 may extend from the ankle to a distal point, such as to a point behind the toes, or in some embodiments, and particularly embodiments in which some or all of the foot portion is flexible, to a point covering some or all of the upper portions of some or all of the toes. It is contemplated that the foot portion 6 may be a sufficient length to impede dorsiflexion. For example, the foot portion 6 may be fitted over an area from the ankle to the metatarsals. In another implementation, the foot portion 6 may be fitted over an area from the ankle to the phalanges. It is further contemplated that the length of the foot portion may be adapted to fit the proportions of the subject.

The heel portion 8 is fitted beneath the heel and is adapted to lift the heel when an anterior force is applied to the leg portion. Lifting the heel may be accomplished by leveraging this force applied to the leg portion 4. For example, when weight is transferred from the proximal region of the foot to the distal region of the foot during ambulation, the leg may push against the leg portion 4 of the shell 2 causing the heel portion 8 to lift the heel. This plantarflexion action may assist a subject in lifting the heel during ambulation, walking, running, bicycling, or other activities requiring motion of the leg. Production of a leveraging force via plantarflexion may be accomplished by providing heel portion 8 shaped to cup the heel from below, as an integral part of shell 2, or as a relatively rigidly attached portion of the shell 2.

The heel portion 8 may be connected to the shell 2 on either the medial or lateral sides. FIGs. 1 and 2 depict an orthosis 1 having a heel portion 8 connected to the shell 2 on the lateral side. The heel portion 8 may cover a sufficient portion of the plantar surface of the heel to facilitate lifting of the heel when a force is applied to the leg portion 4. The heel portion 8 may additionally cover a portion of the posterior surface of the heel to impede slippage of the orthosis during use. The heel portion 8 may be portioned and dimensioned to cup the heel, as shown in FIG. 1.

The leg portion 4 is fitted across the anterior surface of the leg, including for example some or all of the shin. The surface of the leg covered by the leg portion 4 may extend from an anterior portion of the ankle, including some of the upper portion of the foot, to a point below the knee. In one implementation, the leg portion 4 may extend from an anterior portion of the ankle to a point just below the tibial tubercle. It is contemplated that the leg portion 4 may be a sufficient length to provide a pivot for lifting the heel. The leg portion may additionally be fitted to a lateral and/or medial surface of the leg. In particular, the leg portion 4 may be contoured around the lateral and/or medial surface of the leg to prevent slippage of the shell 2 relative to the leg. It is further contemplated that the length of the leg portion may be adapted to fit the proportions of the subject.

The orthosis 1 has a form adapted to permit plantarflexion of the foot. In the implementation shown in FIG. 1, the orthosis is fitted to the heel but otherwise is not fitted to the plantar surface of the foot. This implementation may permit the foot to rotate in plantarflexion. Additionally, this implementation may permit the toes to also plantarflex. This implementation may facilitate the toes to press down against the ground during ambulation creating more leverage for the orthosis and assisting the gait cycle.

The orthosis may further comprise a calf member 10 fitted over a posterior surface of the leg. The calf member may additionally cover a lateral and/or medial surface of the leg. The calf member 10 may be releasably attached to the leg and/or the leg portion 4 of the shell 2. The calf member 10 may impede slippage of the orthosis 1 relative to the leg when in use. In some implementations, the calf member 10 may be molded to the leg of a subject to provide a close fit. The calf member 10 may comprise a flexible or rigid material. In some implementations, the calf member 10 comprises a polymer. In further implementations, the calf member 10 may comprise any or all of low density polyethylene, polypropylene, graphite, carbon fiber and a copolymer mix.

The shell 2 may be a single form and is optionally rigid, semi-rigid, or semi-flexible. In some implementations, the rigidity of the shell 2 will not be consistent throughout all portions of the shell 2. It is contemplated that the degree of rigidity in some or all portions of the shell 2 facilitates the foot portion to selectively impede dorsiflexion of the foot without causing discomfort to the wearer. In particular, it is contemplated that a particular rigidity of the foot portion 6 may be selected to permit dorsiflexion within a range of desired angles. It is further contemplated that rigidity of some or all portions of the shell 2 facilitates desired positioning of the heel portion 8 in conjunction with the leg portion 4 to lift the heel during ambulation. In some implementations, the shell is contoured or molded to the subject's leg and foot. The shell 2 may comprise a polymer. In further implementations, the shell 2 comprises any or all of low density polyethylene, polypropylene, graphite, carbon fiber or a copolymer mix. In order to prevent discomfort for the wearer, some or all of the interior surface(s) of the orthosis may be covered, or otherwise cooperate, with padded material.

The shell 2 may be dimensioned so as to be unobtrusive when worn under clothing or footwear.

The shell 2 may be portioned and dimensioned to a specific range of dorsiflexion of the foot. Inone implementation, the shell 2 may be portioned and dimensioned to an angle of approximately 105°. In an additional implementation, the shell may be portioned and dimensioned to an angle of approximately 95°. In another implementation, the shell 2 may be portioned and dimensioned to an angle of approximately 85°. In a yet further implementation, the shell 2 may be portioned and dimensioned to an angle of approximately 75°.

In some implementations, the shell 2 may be portioned and dimensioned to permit some degree of dorsiflexion. The shell may be portioned and dimensioned to permit dorsiflexion of the foot between 75 and 105 degrees. The shell 2 may be dimensioned such that certain portions of the shell may have different thicknesses. In other implementations, certain portions of the shell 2 may have different rigidities or flexibility. For example, the foot portion 6 may have a thickness such that a specific degree of dorsiflexion is permitted. It is contemplated that the shell 2 may be portioned and dimensioned either in a specific angle of dorsiflexion or in an assortment of dorsiflexion angles to provide the optimal combination of comfort and function for the wearer.

The shell 2 is not necessarily a single form. In some implementations, the foot portion 6 may be connected to the leg portion 18 of the orthosis. For example, the foot portion may be connected to the leg portion 18 with an articulated joint, hinge, or other suitable attachment mechanism (not shown). It is contemplated that the attachment mechanism may accommodate dorsiflexion of the foot through a limited range of angles.

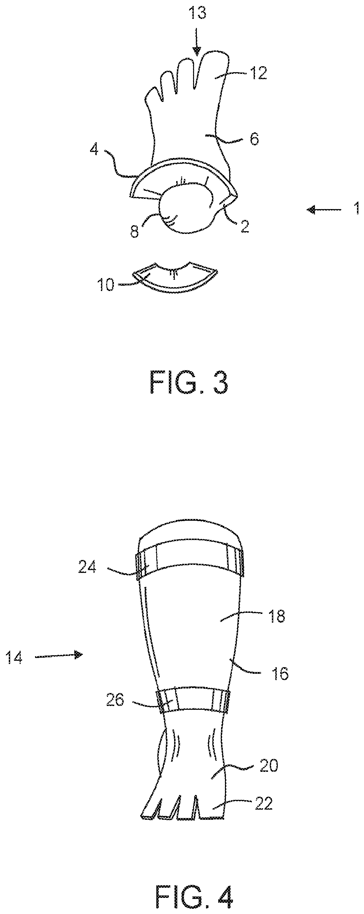

FIG. 3 shows a top view of the orthosis 1. According to the invention, the foot portion 6 of the shell 2 includes toe members 12. The shell 2 has a plurality of toe members 12. The toe members 12 are extensions of the shell 2 that are portioned and dimensioned to be in close proximity to one or more toes. The toe members may impede, limit, or otherwise selectively control dorsiflexion of the phalanges of the respective toes. Some or all of toe members 12 are separated by gaps 13. The gaps 13 may allow the orthosis 1 to be worn with a variety of footwear including but not limited to sandals, flip flops, and athletic footwear.

FIG. 4 shows a front view of another embodiment of an orthosis 14. The orthosis 14 may have a shell 16 that includes a leg portion 18, a foot portion 20, and a heel portion (not shown). The foot portion 20 includes toe members 22. The leg portion 18 may be releasably secured to the leg. Additionally, the calf member (not shown) may be releasably secured to the leg or the leg portion 18 with straps. In FIG. 4, the leg portion 18 is releasably secured to the leg with one or more straps 24, 26, however the means of releasably securing the leg portion to the leg is not limited to straps. The means of releasably securing the leg portion to the leg may include buckles, hook and loop fasteners, laces, belts, buttons, Velcro^{®}, elasticized material, or other fasteners. Similarly, the means of releasably securing the calf member to the leg or the leg portion 18 may include buckles, hook and loop fasteners, laces, belts, buttons, Velcro^{®}, elasticized material or other fasteners.

The many features and advantages of the invention are apparent from the detailed specification and, thus, it is intended by the appended claims to cover all such features and advantages of the invention that fall within the scope of the invention. Further since numerous modifications and changes will readily occur to those skilled in the art, it is not desired to limit the invention to the exact construction and operation illustrated and described, and accordingly all suitable modifications may be resorted to, falling within the scope of the invention.

## Claims

1. An orthosis comprising a shell (2) portioned and dimensioned to be in close proximity to an anatomical foot and leg, said shell (2) comprising:
a leg portion (4) configured to fit over an anterior surface of the leg;
a foot portion (6) configured to fit over a dorsal surface of the foot and adapted to impede dorsiflexion of the foot;
a heel portion (8) configured to fit over a heel of the foot and adapted to lift the heel when an anterior force is applied to the leg portion (4), the heel portion (8) connected to the foot portion (6) on a single side of the orthosis and configured to leave a gap on an opposite side of the orthosis to receive the foot;
wherein the shell (2) is formed in a specific angle of dorsiflexion of the foot; and
wherein the leg portion (4), heel portion (8), and foot portion (6) comprise a single, rigid form; **characterized by** the foot portion (6) comprising
a plurality of toe portions (12) with gaps (13) therebetween, the plurality of toe portions (12) portioned and dimensioned to be in close proximity to a plurality of toes of the foot; and further
wherein only the heel portion (8) of the orthosis is configured to fit over a plantar portion of the foot such that the orthosis permits plantarflexion of the foot.

2. The orthosis of claim 1 wherein the shell (2) is formed by molding the shell (2) to the foot and leg.

3. The orthosis of claim 1 further comprising a means of releasably securing the leg portion (4) to the leg.

4. The orthosis of claim 1 further comprising a calf member (10) configured to fit over the posterior of the leg, wherein the calf member (10) is configured to be releasably secured to the leg.

5. The orthosis of claim 1, the orthosis being adapted to be worn inside footwear.

6. An orthosis comprising a shell (2) portioned and dimensioned to be in close proximity to an anatomical foot and leg, said shell (2) comprising:
a leg portion (4) configured to fit over an anterior surface of the leg and configured for releasably securing to the leg;
a foot portion (6) configured to fit over a dorsal surface of the foot and configured to impede dorsiflexion of the foot;
a heel portion (8) configured to fit over a portion of a plantar surface of the foot and adapted to lift a heel of the foot when a force is applied to the leg portion (4), the heel portion (8) connected to the foot portion (6) on a single side of the orthosis and configured to leave a gap on an opposite side of the orthosis to receive the foot;
wherein the shell (2) is formed in a specific angle of dorsiflexion of the foot; and
wherein the leg portion (4), heel portion (8), and foot portion (6) comprise a single, rigid form; **characterized by** the foot portion (6) comprising
a plurality of toe portions (12) with gaps (13) therebetween, the plurality of toe portions (12) portioned and dimensioned to be in close proximity to a plurality of toes of the foot; and further
wherein only the heel portion (8) of the orthosis is configured to fit over the plantar surface of the foot such that the orthosis permits plantarflexion of the foot.

7. The orthosis of claim 6 further comprising a calf member (10) configured to fit over a posterior of the leg, wherein the calf member (10) is configured to be releasably secured to the leg.

## Patentansprüche

1. Eine Orthese, die eine Schale (2) aufweist, die dahingehend aufgeteilt und dimensioniert ist, sich in unmittelbarer Nähe zu einem anatomischen Fuß und einem anatomischen Bein zu befinden, wobei die Schale (2) folgende Merkmale aufweist:
einen Beinabschnitt (4), der dazu konfiguriert ist, über eine vordere Oberfläche des Beins zu passen;
einen Fußabschnitt (6), der dazu konfiguriert ist, über eine dorsale Oberfläche des Fußes zu passen, und dazu angepasst ist, eine Dorsalflexion des Fußes zu verhindern;
einen Fersenabschnitt (8), der dazu konfiguriert ist, über eine Ferse des Fußes zu passen, und dazu angepasst ist, die Ferse anzuheben, wenn eine vordere Kraft auf den Beinabschnitt (4) ausgeübt wird, wobei der Fersenabschnitt (8) mit dem Fußabschnitt (6) auf einer einzigen Seite der Orthese verbunden ist und dazu konfiguriert ist, einen Zwischenraum auf einer gegenüberliegenden Seite der Orthese zu lassen, um den Fuß aufzunehmen;
wobei die Schale (2) in einem spezifischen Dorsalflexionswinkel des Fußes gebildet ist; und
wobei der Beinabschnitt (4), der Fersenabschnitt (8) und der Fußabschnitt (6) eine einzige, starre Form aufweisen; **dadurch gekennzeichnet, dass** der Fußabschnitt (6) folgende Merkmale aufweist:
eine Mehrzahl von Zehenabschnitten (12) mit Zwischenräumen (13) dazwischen, wobei die Mehrzahl von Zehenabschnitten (12) dahingehend aufgeteilt und dimensioniert ist, sich in unmittelbarer Nähe zu einer Mehrzahl von Zehen des Fußes zu befinden; und ferner
wobei nur der Fersenabschnitt (8) der Orthese dazu konfiguriert ist, über einen plantaren Abschnitt des Fußes zu passen, sodass die Orthese eine Plantarflexion des Fußes ermöglicht.

2. Die Orthese gemäß Anspruch 1, wobei die Schale (2) durch Formen der Schale (2) an den Fuß und das Bein gebildet ist.

3. Die Orthese gemäß Anspruch 1, die ferner eine Einrichtung zum lösbaren Befestigen des Beinabschnitts (4) an dem Bein aufweist.

4. Die Orthese gemäß Anspruch 1, die ferner ein Wadenelement (10) aufweist, das dazu konfiguriert ist, über die Hinterseite des Beins zu passen, wobei das Wadenelement (10) dazu konfiguriert ist, lösbar an dem Bein befestigt zu werden.

5. Die Orthese gemäß Anspruch 1, wobei die Orthese dazu angepasst ist, in Schuhwerk getragen zu werden.

6. Eine Orthese, die eine Schale (2) aufweist, die dahingehend aufgeteilt und dimensioniert ist, sich in unmittelbarer Nähe zu einem anatomischen Fuß und einem anatomischen Bein zu befinden, wobei die Schale (2) folgende Merkmale aufweist:
einen Beinabschnitt (4), der dazu konfiguriert ist, über eine vordere Oberfläche des Beins zu passen, und dazu konfiguriert ist, lösbar an dem Bein befestigt zu werden;
einen Fußabschnitt (6), der dazu konfiguriert ist, über eine dorsale Oberfläche des Fußes zu passen, und dazu konfiguriert ist, eine Dorsalflexion des Fußes zu verhindern;
einen Fersenabschnitt (8), der dazu konfiguriert ist, über einen Abschnitt einer plantaren Oberfläche des Fußes zu passen, und dazu angepasst ist, eine Ferse des Fußes anzuheben, wenn eine Kraft auf den Beinabschnitt (4) ausgeübt wird, wobei der Fersenabschnitt (8) auf einer einzigen Seite der Orthese mit dem Fußabschnitt (6) verbunden ist und dazu konfiguriert ist, einen Zwischenraum auf einer gegenüberliegenden Seite der Orthese zu lassen, um den Fuß aufzunehmen;
wobei die Schale (2) in einem spezifischen Dorsalflexionswinkel des Fußes gebildet ist; und
wobei der Beinabschnitt (4), der Fersenabschnitt (8) und der Fußabschnitt (6) eine einzige, starre Form aufweisen; **dadurch gekennzeichnet, dass** der Fußabschnitt (6) folgende Merkmale aufweist:
eine Mehrzahl von Zehenabschnitten (12) mit Zwischenräumen (13) dazwischen, wobei die Mehrzahl von Zehenabschnitten (12) dahingehend aufgeteilt und dimensioniert ist, sich in unmittelbarer Nähe zu einer Mehrzahl von Zehen des Fußes zu befinden; und ferner
wobei nur der Fersenabschnitt (8) der Orthese dazu konfiguriert ist, über die plantare Oberfläche des Fußes zu passen, sodass die Orthese eine Plantarflexion des Fußes ermöglicht.

7. Die Orthese gemäß Anspruch 6, die ferner ein Wadenelement (10) aufweist, das dazu konfiguriert ist, über eine Hinterseite des Beins zu passen, wobei das Wadenelement (10) dazu konfiguriert ist, lösbar an dem Bein befestigt zu werden.

## Revendications

1. Orthèse comprenant une coque (2) compartimentée et dimensionnée pour être à proximité immédiate d'un pied et d'une jambe anatomiques, ladite coque (2) comprenant :
une partie de jambe (4) configurée pour s'ajuster sur une surface antérieure de la jambe ;
une partie de pied (6) configurée pour s'ajuster sur une surface dorsale du pied et adaptée pour entraver une dorsiflexion du pied ;
une partie de talon (8) configurée pour s'ajuster sur un talon du pied et adaptée pour soulever le talon lorsqu'une force antérieure est exercée à la partie de jambe (4), la partie de talon (8) étant reliée à la partie de pied (6) sur un seul côté de l'orthèse et configurée pour laisser un espace sur un côté opposé de l'orthèse pour recevoir le pied ;
dans laquelle la coque (2) est formée dans un angle spécifique de dorsiflexion du pied ; et
dans laquelle la partie de jambe (4), la partie de talon (8) et la partie de pied (6) comprennent une seule et même forme rigide ; **caractérisée en ce que** la partie de pied (6) comprend
une pluralité de parties d'orteils (12) avec des espaces (13) entre elles, la pluralité des parties d'orteils (12) compartimentée et dimensionnée pour être en proximité immédiate à une pluralité d'orteils du pied ; et en outre
dans laquelle seule la partie de talon (8) de l'orthèse est configurée pour s'ajuster sur une partie plantaire du pied, de sorte que l'orthèse permette la flexion plantaire du pied.

2. Orthèse selon la revendication 1, dans laquelle la coque (2) est formée par moulage de la coque (2) au pied et à la jambe.

3. Orthèse selon la revendication 1, comprenant en outre un moyen permettant de fixer de manière amovible la partie de jambe (4) à la jambe.

4. Orthèse selon la revendication 1, comprenant en outre un élément de mollet (10) configuré pour s'ajuster sur la partie postérieure de la jambe, dans laquelle l'élément de mollet (10) est configuré pour être fixé de manière amovible à la jambe.

5. Orthèse selon la revendication 1, l'orthèse étant adaptée pour être portée à l'intérieur d'une chaussure.

6. Orthèse comprenant une coque (2) compartimentée et dimensionnée pour être à proximité immédiate d'un pied et d'une jambe anatomiques, ladite coque (2) comprenant :
une partie de jambe (4) configurée pour s'ajuster sur une surface antérieure de la jambe et configurée pour être fixée de manière amovible à la jambe ;
une partie de pied (6) configurée pour s'ajuster sur une surface dorsale du pied et configurée pour entraver une dorsiflexion du pied ;
une partie de talon (8) configurée pour s'ajuster sur une partie d'une surface plantaire du pied et adaptée pour soulever un talon du pied lorsqu'une force est exercée à la partie de jambe (4), la partie de talon (8) étant reliée à la partie de pied (6) sur un seul côté de l'orthèse et configurée pour laisser un espace sur un côté opposé de l'orthèse pour recevoir le pied ;
dans laquelle la coque (2) est formée dans un angle spécifique de dorsiflexion du pied ; et
dans laquelle la partie de jambe (4), la partie de talon (8) et la partie de pied (6) comprennent une seule et même forme rigide ; **caractérisée en ce que** la partie de pied (6) comprend
une pluralité de parties d'orteils (12) avec des espaces (13) entre elles, la pluralité des parties d'orteils (12) compartimentée et dimensionnée pour être en proximité immédiate à une pluralité d'orteils du pied ; et en outre
dans laquelle seule la partie de talon (8) de l'orthèse est configurée pour s'ajuster sur la surface plantaire du pied, de sorte que l'orthèse permette la flexion plantaire du pied.

7. Orthèse selon la revendication 6, comprenant en outre un élément de mollet (10) configuré pour s'ajuster sur une partie postérieure de la jambe, dans laquelle l'élément de mollet (10) est configuré pour être fixé de manière amovible à la jambe.
